(19) Europäisches Patentamt
European Patent Office
Office européen des brevets

(11) Veröffentlichungsnummer: **0 045 005**
**B1**

(12) # EUROPÄISCHE PATENTSCHRIFT

(45) Veröffentlichungstag der Patentschrift: **11.07.84**

(51) Int. Cl.³: **C 07 D 277/40**

(21) Anmeldenummer: **81105524.3**

(22) Anmeldetag: **14.07.81**

(54) **Verfahren zur Herstellung von 2-(2-Aminothiazol-4-yl)-2-(syn)-methoxyiminoessigestern.**

(30) Priorität: **24.07.80 CH 5658/80**

(43) Veröffentlichungstag der Anmeldung:
**03.02.82 Patentblatt 82/5**

(45) Bekanntmachung des Hinweises auf die Patenterteilung:
**11.07.84 Patentblatt 84/28**

(84) Benannte Vertragsstaaten:
**AT BE CH DE FR GB IT LI LU NL SE**

(56) Entgegenhaltungen:
**DE - A - 2 818 025**

**PATENT ABSTRACTS OF JAPAN, unexamined applications, Section C, Band 1, Nr. 131, 28. Oktober 1977**
**THE PATENT OFFICE JAPANESE GOVERNMENT, Seite 2943, C 77**
**Kontakte(Merck) 3/76, S. 5 und 6**
**Kontakte(Merck) 3/80, S. 21**

**Die Akte enthält technische Angaben, die nach dem Eingang der Anmeldung eingereicht wurden und die nicht in dieser Patentschrift enthalten sind.**

(73) Patentinhaber: **LONZA AG, Gampel/Wallis (CH)**

(72) Erfinder: **Huwiler, Alfred, Dr., Sandstrasse 5, VISP, (Kanton Wallis) (CH)**
Erfinder: **Tenud, Leander, Dr., Balfrinstrasse 23, VISP, (Kanton Wallis) (CH)**

(74) Vertreter: **von Füner, Alexander, Dr. et al, Patentanwälte v. Füner, Ebbinghaus, Finck Mariahilfplatz 2 & 3, D-8000 München 90 (DE)**

**EP 0 045 005 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

ACTORUM AG

**Beschreibung**

Die Erfindung betrifft die Herstellung von 2-(2-Aminothiazol-4-yl)-2-(syn)methoxyiminoessigester, welcher zusammen mit seinem Derivaten Seitenketten semisynthetischer Cephalosporine der dritten Generation bildet. Als solche sind diese Substanzen von zunehmender Wichtigkeit.

Bei den bisher bekannten Herstellungsverfahren kann zwischen drei Synthesewegen unterschieden werden: Aus den deutschen Offenlegungsschriften Nrn. 2812625 und 2831332 bzw. den französischen Patentanmeldungen Nrn. 2390442 und 2384781 hat man Kenntnis von folgendem Syntheseweg: Der Acetessigester wird oximiert, halogeniert und mit Thioharnstoff zum Hydroxyiminothiazolessigester umgesetzt. Dieser wird dann mit Diazomethan bzw. Dimethylsulfat, ohne die Anwendung der Methode der Phasentransferkatalyse, in den Methoxyiminothiazolylessigester übergeführt.

Die deutschen Offenlegungsschriften Nrn. 2715385 und 2805655 offenbaren nachstehendes Vorgehen: Der Oxyiminoacetessigester wird methyliert, halogeniert und dann mit Thioharnstoff umgesetzt.

Der Oxyiminoacetessigester wird zuerst halogeniert, dann methyliert und nachher mit Thioharnstoff zum Methoxyiminothiazolylessigester umgesetzt. Diese Arbeitsmethode ist der deutschen Offenlegungsschrift Nr. 2806226 und den französischen Patentanmeldungen Nrn. 2381053 und 2384779 zu entnehmen.

Bei allen diesen Synthesewegen fällt ein Gemisch von syn- und anti-Produkt an:

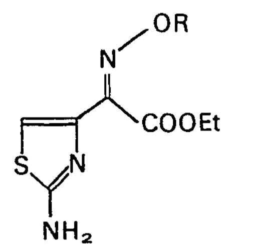

syn

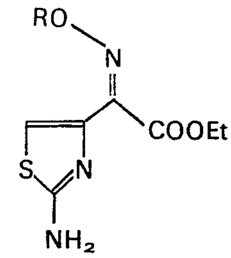

anti

Für die Anwendung im Zusammenhang mit semisynthetischen Cephalosporinen, d.h. für die Umsetzung mit 7-Aminocephalosporansäure, ist aber nur das syn-Isomere erwünscht.

Aufgabe der Erfindung ist es, den erwähnten Nachteil zu vermeiden.

Erfindungsgemäss wurde dies durch ein Verfahren gemäss den Patentansprüchen erreicht, welches die nachstehende Reaktionsfolge aufweist:

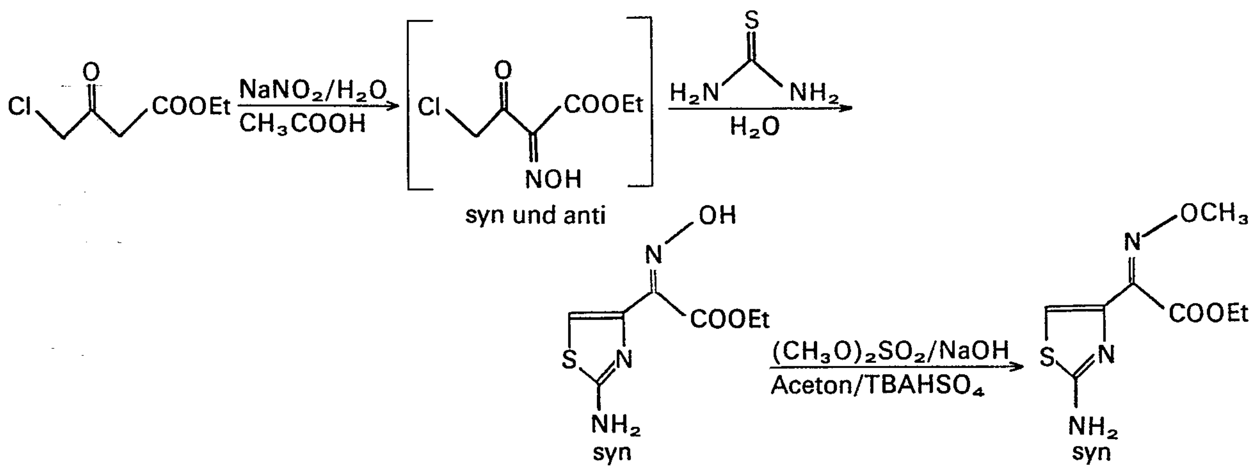


Das erfindungsgemässe Verfahren weist gegenüber den bekannten Verfahren folgende Vorteile auf:

1. Das erfundene Verfahren liefert nur das gewünschte syn-Produkt in isomerenreiner Form.

2. Der Hydroxyiminothiazolylessigester kann in einem Schritt, ohne Isolation des Zwischenproduktes, hergestellt werden.

3. Die Methylierung des Hydroxyiminothiazolylessigesters erfolgt mittels Phasentransferkatalyse. Dadurch kann die Ausbeute in dieser Stufe von 40 bis 50 auf 80% gesteigert werden.

Das Verfahren wird vorzugsweise derart ausgeführt, dass man in einer ersten Phase 4-Chloracetessigester zusammen mit Eisessig vorlegt, auf 0° C abkühlt und mit Alkalinitrit, vorzugsweise mit Natriumnitrit, oximiert.

Die Oximierung hat unter Kühlung zu erfolgen, so dass die Temperatur im Reaktionsmedium nach beendeter Zudosierung etwa −15° C beträgt.

Bei dieser Temperatur wird dann der gebildete 4-Chlor-2-hydroxyiminoacetessigester, ohne ihn zu isolieren, mit Thioharnstoff direkt in den 2-(2-Aminothiazol-4-yl)-2-(syn)hydroxyiminoessigester übergeführt.

Der Thioharnstoff liegt vorzugsweise in Wasser gelöst vor. Zweckmässig wird das Zusetzen des 4-Chlor-2-hydroxyiminoacetessigesters zur Thioharnstofflösung so durchgeführt, dass die Reaktionstemperatur nicht über +40° C steigt.

In einer zweiten Phase wird zweckmässig der 2-(2-Aminothiazol-4-yl)-2-(syn)hydroxyiminoessigester zusammen mit einem Phasentransferkatalysator in einem organischen Lösungsmittel suspendiert, mit Natronlauge versetzt und mit Dimethylsulfat zum 2-(2-Aminothiazol-4-yl)-2-(syn)methoxyiminoessigester methyliert. Die bevorzugte Reaktionstemperaturen in dieser zweiten Phase liegen bei 0° C.

Als Phasentransferkatalysatoren können quaternäre Ammonium- oder Phosphoniumsalze eingesetzt werden, vorzugsweise aber Tetrabutylammoniumhydrogensulfat.

Als organische Lösungsmittel können neben dem vorzugsweise eingesetzten Aceton aprotische, mit Wasser mischbare oder mit Wasser nicht mischbare organische Lösungsmittel verwenet werden.

*Beispiel:*

*Phase I*

*Herstellung von 2-(2-Aminothiazol-4-yl)-2-(syn)hydroxyiminoessigester*

174,0 g (1,0 mol) 4-Chloracetessigester und 176,0 g Eisessig wurden in einem 500-ml-Sulfierkolben vorgelegt und auf 0° C abgekühlt. Zu dieser Lösung tropfte man während 45 min eine Lösung von 72,8 g 99%igem Natriumnitrit (1,05 mol) in 102,0 g Wasser unter Kühlung so zu, dass die Innentemperatur langsam absank und nach beendeter Zudosierung −15° C erreichte. Die so erhaltene dunkelrote Lösung von 4-Chlor-2-hydroxyiminoacetessigester wurde dann noch 2 h bei dieser Temperatur gerührt. In dieser Zeit wurde in einem 1500-ml-Sulfierkolben eine Lösung von 76,1 g (1,0 mol) Thioharnstoff in 610,0 g Wasser vorbereitet und auf 30 bis 35° C erwärmt. In diese letztere Lösung wurde dann mittels einer Dosierpumpe die immer noch eine Temperatur von −15° C aufweisende Lösung von 4-Chlor-2-hydroxyiminoacetessigester während 45 min so eingeleitet, dass die Reaktionstemperatur langsam auf +40° C kletterte, diese aber nie überstieg. Nach beendeter Zudosierung wurde noch 4 h gerührt. Dabei fiel der 2-(2-Aminothiazol-4-yl)-2-(syn)hydroxyiminoessigester langsam aus. Der Ester wurde anschliessend abfiltriert, mit 1 l Wasser gewaschen und im Vakuum von 1,467 kPa (11 Torr/Hg) im Trockenschrank bei 50° C über Nacht getrocknet.

So wurden 144,2 g gebliche Kristalle des 2-(2-Aminothiazol-4-yl)-2-(syn)hyroxyiminoesters mit enem Smp. = Fp. von 185 bis 186° C erhalten. Die Ausbeute betrug 67%, bezogen auf 4-Chloracetessigester.

Das erhaltene Produkt wies syn-Konfiguration auf (NMR[DMSO-$d_6$, 60 MHz]: 11,7 ppm [N−OH]; 6,9 ppm [Proton des Thiazolrings]).

*Phase II*

10,7 g (48 mmol) 2-(2-Aminothiazol-4-yl)-2-(syn)hydroxyiminoessigester und 1,7 g (5 mmol) Tetrabutylammoniumhydrogensulfat wurden in 56,0 g Aceton in einem Dreihalskolben mit 250 ml Inhalt vorgelegt und auf 0° C abgekühlt.

Zu dieser Suspension wurden innerhalb weniger Minuten 5,0 g 50%ige Natronlauge zugegeben und die so erhaltene Mischung während 30 min bei 5° C gerührt. Dann wurde innerhalb 30 min eine Lösung von 7,6 g (60 mmol) Dimethylsulfat in Aceton zugetropft, das Reaktionsgemisch während weiterer 3 h bei 0° C gerührt und von ungelösten Anteilen durch Filtrieren befreit. Durch Eindampfen des Filtrates im Vakuum von 1,467 kPa (11 Torr/Hg) bei ca. 30° C erhielt man einen Rückstand, welcher in 150 g Wasser aufgeschlämmt, abgenutscht und über Nacht im Vakuum von 1,467 kPa (11 Torr/Hg) in einem Trockenschrank bei 50° C getrocknet wurde.

Auf diese Weise erhielt man 8,9 g bräunliche Kristalle des 2-(2-Aminothiazol-4-yl)-2-(syn)methoxyiminoessigesters mit einem Smp. = Fp. von 155 bis 157° C.

Dies entspricht einer Ausbeute von 80%, bezogen auf den 2-(2-Aminothiazol-4-yl)-2-(syn)hydroxyiminoessigester.

Das erhaltene Produkt wies syn-Konfiguration auf (NMR[DMSO-$d_6$, 60 MHz]: 6,95 ppm [Proton des Thiazolrings]; 3,9 ppm [N−$OCH_3$]).

## Patentansprüche

1. Verfahren zur Herstellung von 2-(2-Aminothiazol-4-yl)-2-(syn)methoxyiminoessigestern, dadurch gekennzeichnet, dass man 4-Chloracetessigester oximiert, den entstandenen Chlorhydroxyiminoester ohne Isolierung desselben direkt mit Thioharnstoff zum 2-(2-Aminothiazol-4-yl)-2-(syn)hydroxyiminoessigester umsetzt und letzteren unter Phasentransferkatalyse mit Dimethylsulfat methyliert.

2. Verfahren gemäss Patentanspruch 1, dadurch gekennzeichnet, dass die Oximierung des 4-Chloracetessigesters mit Alkalinitrit in Eisessig bei einer Anfangstemperatur von 0° C durchgeführt wird.

3. Verfahren gemäss den Patentansprüchen 1 und 2, dadurch gekennzeichnet, dass die bei der Oximierung erhaltene Lösung, die eine Temperatur von unter 0° C aufweist, direkt in eine wässerige Thioharnstofflösung so eingeleitet wird, dass die reaktionstemperatur nicht über +40° C steigt.

4. Verfahren gemäss den Patentansprüchen 1 bis 3, dadurch gekennzeichnet, dass der erhaltene 2-(2-Aminothiazol-4-yl)-2-(syn)hydroxyiminoessigester mittels einer Phasentransferkatalyse mit Dimethylsulfat bei Temperaturen zwischen 0 und +5° C umgesetzt wird.

5. Verfahren gemäss Patentanspruch 4, dadurch gekennzeichnet, dass der 2-(2-Aminothiazol-4-yl)-2-(syn)hydroxyiminoessigester zusammen mit Tetrabutylammoniumhydrogensulfat in Aceton suspendiert wird und, nach Versetzen dieser Suspension mit Alkalilauge, durch Zuführen von Dimethylsulfat in Aceton methyliert wird.

## Claims

1. Process for the preparation of 2-(2-aminothiazol-4-yl)-2-(syn)methoxyiminoacetic esters, characterised in that one oximates 4-chloroacetoacetic ester, reacts the resultant chlorohydroxyimino ester, without isolation thereof, directly with thiourea to give 2-(2-aminothiazol-4-yl)-2-(syn)hydroxyiminoacetic ester and methylates the latter with dimethyl sulphate with phase transfer catalysis.

2. Process according to Claim 1, characterised in that the oximation of the 4-chloroacetoacetic ester is carried out with alkali metal nitrite in glacial acetic acid at a starting temperature of 0° C.

3. Process according to Claim 1 and 2, characterised in that the solution obtained in the case of the oximation, which has a temperature of below 0° C, is introduced directly into an aqueous thiourea solution in such a manner that the reaction temperature does not increase above +40° C.

4. Process according to Claims 1 to 3, characterised in that the 2-(2-aminothiazol-4-yl)-2-(syn)hydroxyiminoacetic ester obtained is reacted by means of a phase transfer catalysis with dimethyl sulphate at temperatures between 0 and +5° C.

5. Process according to Claim 4, characterised in that the 2-(2-aminothiazol-4-yl)-2-(syn)hydroxyiminoacetic ester is suspended in acetone, together with tetrabutylammonium hydrogen sulphate, and, after mixing this suspension with an aqueous solution of an alkali metal hydroxide, is methylated by the introduction of dimethyl sulphate in acetone.

## Revendications

1. Procédé de préparation d'esters 2-(2-aminothiazol-4-yl)-2-(syn)methoxyimino-acétiques, caractérisé en ce que l'on oxime un ester 4-chloroacétylacétique, en ce que l'on convertit directement l'ester chlorhydroxyiminé formé, sans l'isoler en ester 2-(2-aminothiazol-4-yl)-2-(syn)hydroxyiminoacétique par réaction avec la thio-urée et en ce que l'on méthyle ce dernier par le sulfate de diméthyle avec catalyse à transfert de phase.

2. Procédé selon la revendication 1, caractérisé en ce que l'oximation de l'ester 4-chloracétylacétique par un nitrite alcalin est effectuée dans l'acide acétique glacial à une température initiale de 0° C.

3. Procédé selon les revendications 1 et 2, caractérisé en ce que la solution obtenue lors de l'oximation, qui présente une température inférieure à 0° C, est injectée directement dans une solution aqueuse de thio-urée dans des conditions telles que la température de réaction ne dépasse pas +40° C.

4. Procédé selon les revendications 1 à 3, caractérisé en ce que l'on fait réagir l'ester 2-(2-aminothiazol-4-yl)-2-(syn)hydroxyiminoacétique obtenu, avec le sulfate de diméthyle à des températures entre 0 et +5° C par catalyse à transfert de phase.

5. Procédé selon la revendication 4, caractérisé en ce que l'on met l'ester 2-(2-aminothiazol-4-yl)-2-(syn)hydroxyiminoacétique ensemble avec le bisulfate de tétrabutylammonium en suspension dans l'acétone et, après avoir ajouté une lessive alcaline à cette suspension, en ce que l'on méthyle dans l'acétone par addition de sulfate de diméthyle.